# NEUE EUROPÄISCHE PATENTSCHRIFT

(11) **EP 1 926 754 B2**
(45) Veröffentlichungstag und Bekanntmachung des Hinweises auf die Entscheidung über den Einspruch: **22.12.2021**
(45) Hinweis auf die Patenterteilung: 05.06.2013
(21) Anmeldenummer: 06793089.1
(22) Anmeldetag: 31.08.2006
(51) Int. Cl.: C08F 2/01, A61L 15/60, B01J 19/26, C08F 2/04, C08F 2/44, A61L 15/24, C08K 5/00

(54) **POLYMERISATIONSVERFAHREN**
POLYMERIZATION METHOD
PROCEDE DE POLYMERISATION

(30) Priorität: 07.09.2005 DE 102005042608
(43) Veröffentlichungstag der Anmeldung: 04.06.2008
(73) Patentinhaber: BASF SE, 67056 Ludwigshafen am Rhein (DE)
(72) Erfinder: WEISMANTEL, Matthias, 63637 Jossgrund (DE); DE MARCO, Michael, 69469 Weinheim (DE); DAISS, Andreas, 68165 Mannheim (DE); VAN ESBROECK, Dominicus, NL-4725 SN Wouse Plantage (NL); POSSEMIERS, Karl, J., B-2900 Schoten (BE); DE KAEY, Ronny, B-2531 Vremde (BE); VAN MIERT, Leo, B-2950 Kapellen (BE)
(74) Vertreter: BASF IP Association
(86) Internationale Anmeldenummer: PCT/EP2006/065846
(87) Internationale Veröffentlichungsnummer: WO 2007/028749

(56) Entgegenhaltungen:
- EP-A1- 0 068 159
- EP-A1- 0 100 423
- EP-A1- 0 574 260
- EP-A1- 0 780 424
- EP-A1- 1 470 905
- EP-A2- 0 312 952
- EP-A2- 0 315 185
- EP-A2- 0 349 241
- EP-A2- 0 955 086
- EP-A2- 1 418 000
- WO-A1-03/022896
- WO-A1-03/051415
- WO-A1-03/051939
- WO-A1-03/051940
- WO-A1-03/053482
- WO-A1-2004/099265
- WO-A1-2006/109845
- WO-A2-02/32964
- DE-A1- 3 540 994
- GB-A- 837 974
- GB-A- 1 255 764
- JP-A- H0 427 291
- JP-A- S5 632 514
- JP-A- H11 240 903
- JP-A- S55 133 413
- JP-A- 2000 038 407
- JP-A- 2005 162 834
- US-A- 4 402 914
- "Modern Superabsorbent Polymer Technology", 1998, F.L.Buchholz Wiley-VCH Verlag pages 19-117,
- "B. Copolymerization with a crosslinking agent D. Self-crosslinking polymerization" In: "Superabsorbent Polymer, The Society of Polymer Science", 15 November 1987 (1987-11-15) pages 37-40,46-47,

## Beschreibung

Die vorliegende Erfindung betrifft ein Verfahren zur kontinuierlichen Herstellung wasserabsorbierender Polymere, wobei eine Monomerlösung mit mindestens einem Vernetzer vermischt und die erhaltene Mischung polymerisiert wird, wobei die Verweilzeit der Mischung zwischen der Zugabe des mindestens einen Vernetzers und dem Eintritt in den Polymerisationsreaktor weniger als 180 Sekunden beträgt.

Wasserabsorbierende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, wobei wasserabsorbierende Polymere auf Basis teilneutralisierter Acrylsäure bevorzugt werden. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Herstellung der wasserabsorbierenden Polymere wird beispielsweise in der Monographie "Modem Superabsorbent Polymer Technology", F.L. Buchholz und A.T. Graham, Wiley-VCH, 1998, oder in Ullmann's Encyclopedia of Industrial Chemistry, 6. Auflage, Band 35, Seiten 73 bis 103, beschrieben. Das bevorzugte Herstellungsverfahren ist die Lösungs- oder Gelpolymerisation. Bei dieser Technologie wird zunächst eine Monomermischung hergestellt, die diskontinuierlich neutralisiert und dann in einen Polymerisationsreaktor überführt wird, oder bereits im Polymerisationsreaktor vorgelegt wird. Im sich anschließenden diskontinuierlichen oder kontinuierlichen Verfahren erfolgt die Reaktion zum Polymergel, das im Falle einer gerührten Polymerisation bereits zerkleinert wird. Das Polymergel wird anschließend getrocknet, gemahlen und gesiebt und dann zur weiteren Oberflächenbehandlung transferiert.

Ein kantinuierliches Polymerisationsverfahren liegt beispielsweise der WO-A-01/38402 zugrunde, wobei die wässrige Monomerlösung zusammen mit dem Initiator und dem Inertgas kontinuierlich einem Mischkneter mit mindestens zwei achsparallel rotierenden Wellen zugeführt wird.

Kontinuierliche Gelpolymerisationen sind weiterhin bekannt aus WO-A-03/004237, WO-A-03/022896 und WO-A-01/016197.

Aufgabe der vorliegenden Erfindung war die Bereitstellung eines verbesserten Polymerisationsverfahrens zur Herstellung vernetzter Polymere, wobei der Vemetzerumsatz möglichst hoch und der Anteil unvernetzter Polymere möglichst niedrig sein sollte.

Gelöst wurde die Aufgabe durch ein Verfahren zur kontinuierlichen Herstellung wasserabsorbierender Polymere, wobei eine Monomerlösung mit mindestens einem Vernetzer vermischt und die erhaltene Mischung polymerisiert wird, dadurch gekennzeichnet, dass die Verweilzeit der Mischung zwischen der Zugabe des mindestens einen Vernetzers und dem Eintritt in den Polymerisationsreaktor mindestens eine Sekunde und weniger als 60 Sekunden beträgt.

Die Verweilzeit der Mischung zwischen der Zugabe des mindestens einen Vernetzers und dem Eintritt in den Polymerisationsreaktor beträgt vorzugsweise weniger als 30 Sekunden, besonders bevorzugt weniger als 10 Sekunden. Ganz besonders vorteilhaft ist eine Verweilzeit im Bereich von 1 bis 5 Sekunden.

Die Polymerisationsneigung läßt sich vermindern, wenn die Verbindung zwischen Vernetzerdosierung und Polymerisationsreaktor zumindest teilweise, vorzugsweise zumindest zu mindestens 50% der Fläche, besonders bevorzugt soweit konstruktiv möglich vollständig, eine Materialoberfläche aufweist, die gegenüber Wasser einen Kontaktwinkel von mindestens 60°, vorzugsweise mindestens 90°, besonders bevorzugt mindestens 100°, hat.

Der Kontaktwinkel ist ein Maß für das Benetzungsverhalten und wird gemäß DIN 53900 gemessen.

Geeignete Materialien mit entsprechendem Benetzungsverhalten sind Polyethylen, Polypropylen, Polyester, Polyamid, Polytetrafluorethylen, Polyvinylchlorid, Epoxidharze und Silikonharze. Ganz besonders bevorzugt ist Polypropylen.

Besonders vorteilhaft ist das erfindungsgemäße Verfahren, wenn der Vernetzer nach Art und Menge in der Monomerlösung nicht vollständig löslich ist und in der Monomerlösung zumindest teilweise dispergiert vorliegt. Das Vorliegen einer Dispersion kann durch Streulichtmessungen leicht ermittelt werden.

Die Viskosität der Monomerlösung beträgt bei 15°C vorzugsweise von 5 bis 200 mPas, besonders bevorzugt von 10 bis 100 mPas, ganz besonders bevorzugt von 20 bis 50 mPas, wobei die Viskosität mit einem Brookfield-Viskosimeter (Spindel 2, 100 Upm) gemessen wird.

Die Monomerkonzentration in der Monomerlösung beträgt vorzugsweise von 10 bis 80 Gew.-%, besonders bevorzugt von 20 bis 60 Gew.-%, ganz besonders bevorzugt von 30 bis 50 Gew.-%.

Die Monomerlösung enthält mindestens ein einfach ethylenisch ungesättigtes Monomer, vorzugsweise Acrylsäure und/oder deren Salze. Der Anteil an Acrylsäure und/oder deren Salze an der Gesamtmonomermenge beträgt vorzugsweise mindestens 50 mol-%, besonders bevorzugt mindestens 90 mol-%, ganz besonders bevorzugt mindestens 95 mol-%.

In einer bevorzugten Ausführungsform der vorliegenden Erfindung wird der mindestens eine Vernetzer über ein Venturi-Rohr dosiert.

Ein Venturi-Rohr ist eine in ihrer Länge begrenzte Rohrverengung, in der der Druckverlust weitgehend reversibel in kinetische Energie umgewandelt wird. Dazu wird die Querschnittsfläche F₁ auf der Strecke L₁ (Einlaufstrecke) auf den Querschnitt F₂ vermindert, der Querschnittsfläche F₂ wird auf der Strecke L₂ (Einschnürzone) konstant gehalten und anschließend wird die Querschnittsfläche F₂ auf der Strecke L₃ (Diffusor) wieder auf die Querschnittsfläche F₁ geweitet. Dabei ist die Querschnittsfläche F₁ größer als die Querschnittsfläche F₂ und die Länge L₃ größer als die Länge L₁.

Die Dosierung des Vernetzers erfolgt vorzugsweise im Bereich der Strecke L₁ oder der Strecke L₂.

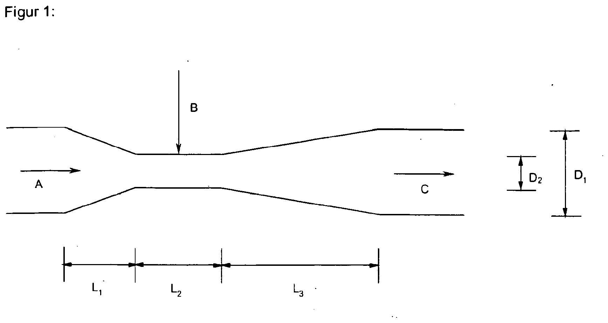

Die Figur 1 zeigt ein typisches Venturi-Rohr, wobei die Bezugszeichen die folgende Bedeutung haben:

| | |
|---|---|
| A: | Monomerlösung vor Vernetzerdosierung |
| B: | Vernetzerzuführung |
| C: | Monomerlösung mit Vernetzer |
| L₁: | Einlaufstrecke |
| L₂: | Einschnürzone |
| L₃: | Diffusor |
| D₁: | Durchmesser der Rohrleitung |
| D₂: | Durchmesser der Einschnürzone |

Die optimale Auslegung eines Venturi-Rohrs ist dem Fachmann an sich bekannt. Vorzugsweise wird das Venturi-Rohr so ausgelegt, dass der Druck im Bereich der Strecke L₂ weniger als der Umgebungsdruck beträgt (Saugförderung) und/oder das die Strömung im Bereich der Strecke L₂ turbulent ist, wobei die Reynolds-Zahl mindestens 1000, vorzugsweise mindestens 2000, besonders bevorzugt mindestens 3000, ganz besonders bevorzugt mindestens 4000, und üblicherweise weniger als 10.000.000 betragen sollte.

Die Dosierung des mindestens einen Vernetzers kann über eine oder mehrere Zugabestellen erfolgen.

Beispielsweise können die Edukte über zwei, drei, vier, fünf oder sechs Zugabestellen dosiert werden, wobei die Zugabestellen vorzugsweise so angeordnet sind, dass sie eine gemeinsame Achse aufweisen (für zwei Zugabestellen) oder einen symmetrischen Stern bilden (für mindestens drei Zugabestellen) und die Achse bzw. Stern senkrecht zur Flussrichtung der Monomerlösung befindet (Mehrfachzugabestellen).

Das Aufteilen in mehrere Zugabestellen bewirkt eine gleichmäßigere Durchmischung.

Bei Verwendung mehrerer Vernetzer können diese getrennt oder als Mischung dosiert werden.

Es ist auch möglich den mindestens einen Vernetzer erst mit einer Teilmenge der Monomerlösung zu mischen und anschließend diese Mischung mit der Hauptmenge der Monomerlösung zu mischen.

Vorzugsweise wird eine vorneutralisierte Monomerlösung mit dem mindestens einen Vernetzer gemischt, die Mischung inertisiert, die inertisierte Mischung mit einem Initiator vermischt und polymerisiert.

Die wasserabsorbierenden Polymere werden beispielsweise durch Polymerisation einer Monomerlösung, enthaltend
a) mindestens ein ethylenisch ungesättigtes, säuregruppentragendes Monomer,
b) mindestens einen Vernetzer,
c) gegebenenfalls ein oder mehrere mit dem Monomeren a) copolymerisierbare ethylenisch und/oder allylisch ungesättigte Monomere und
d) gegebenenfalls ein oder mehrere wasserlösliche Polymere, auf die die Monomere a), b) und ggf. c) zumindest teilweise aufgepfropft werden können,
erhalten.

Geeignete Monomere a) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Besonders bevorzugte Monomere sind Acrylsäure und Methacrylsäure. Ganz besonders bevorzugt ist Acrylsäure.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden 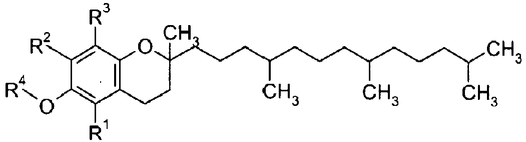 wobei R¹ Wasserstoff oder Methyl, R² Wasserstoff oder Methyl, R³ Wasserstoff oder Methyl und R⁴ Wasserstoff oder ein Säurerest mit 1 bis 20 Kohlenstoffatomen bedeutet.

Bevorzugte Reste für R⁴ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R¹ = R² = R³ = Methyl, insbesondere racemisches alpha-Tocopherol. R¹ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A-0 530 438 beschrieben, Di- und Triacrylate, wie in EP-A-0 547 847, EP-A-0 559 476, EP-A-0 632 068, WO-A-93/21237, WO-A-03/104299, WO-A-03/104300, WO-A-03/104301 und DE-A-103 31 450 beschrieben, gemischte Acrylate, die neben Acrylatgruppen weitere ethylenisch ungesättigte Gruppen enthalten, wie in DE-A-103 31 456 und WO-A-04/013064 beschrieben, oder Vernetzermischungen, wie beispielsweise in DE-A-195 43 368, DE-A-196 46 484, WO-A-90/15830 und WO-A-02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. - methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO-A-03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3-bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf. Die Menge an Vernetzer b) beträgt vorzugsweise 0,01 bis 1 Gew.-%, besonders bevorzugt 0,05 bis 0,5 Gew.-%, ganz besonders bevorzugt 0,1 bis 0,3 Gew.-%, jeweils besorgen auf das Monomer a).

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die bevorzugten Polymerisationsinhibitoren benötigen für eine optimale Wirkung gelösten Sauerstoff. Üblicherweise werden die Monomerlösungen vor der Polymerisation weitgehend von Sauerstoff befreit (Inertisierung), beispielsweise mittels Durchströmen mit einem inerten Gas, vorzugsweise Stickstoff. Dadurch werden die Polymerisationsinhibitoren in ihrer Wirkung deutlich abgeschwächt. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Herstellung eines geeigneten Grundpolymers sowie weitere geeignete hydrophile ethylenisch ungesättigte Monomere d) werden in DE-A-199 41 423, EP-A-0 686 650, WO-A-01/45758 und WO-A-03/104300 beschrieben.

Wasserabsorbierende Polymere werden üblicherweise durch Polymerisation einer wässrigen Monomerlösung und gegebenenfalls einer anschließenden Zerkleinerung des Hydrogels erhalten. Geeignete Herstellverfahren sind in der Literatur beschrieben. Wasserabsorbierende Polymere können beispielsweise erhalten werden durch
- Gelpolymerisation im Batchverfahren bzw. Rohrreaktor und anschließender Zerkleinerung im Fleischwolf, Extruder oder Kneter (EP-A-0 445 619, DE-A-19 846413)
- Polymerisation im Kneter, wobei durch beispielsweise gegenläufige Rührwellen kontinuierlich zerkleinert wird, (WO-A-01/38402)
- Polymerisation auf dem Band und anschließende Zerkleinerung im Fleischwolf, Extruder oder Kneter (DE-A-38 25 366, US-6,241,928)
- Emulsionspolymerisation, wobei bereits Perlpolymerisate relativ enger Gelgrößenverteilung anfallen (EP-A-0 457 660)
- In-situ Polymerisation einer Gewebeschicht, die zumeist im kontinuierlichen Betrieb zuvor mit wässriger Monomerlösung besprüht und anschließend einer Photopolymerisation unterworfen wurde (WO-A-02/94328, WO-A-02/94329)

Die Umsetzung wird vorzugsweise in einem Kneter, wie beispielsweise in WO-A-01/38402 beschrieben, oder auf einem Bandreaktor, wie beispielsweise in EP-A-0 955 086 beschrieben, durchgeführt.

Die Neutralisation kann auch teilweise nach der Polymerisation auf der Stufe des Hydrogels durchgeführt werden. Es ist daher möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Hydrogels eingestellt wird. Die Monomerlösung kann durch Einmischen des Neutralisationsmittels neutralisiert werden. Das Hydrogel kann mechanisch zerkleinert werden, beispielsweise mittels eines Fleischwolfes, wobei das Neutralisationsmittel aufgesprüht, übergestreut oder aufgegossen und dann sorgfältig untergemischt werden kann. Dazu kann die erhaltene Gelmasse noch mehrmals zur Homogenisierung gewolft werden. Die Neutralisation der Monomerlösung auf den Endneutralisationsgrad ist bevorzugt.

Das neutralisierte Hydrogel wird dann mit einem Band- oder Walzentrockner getrocknet bis der Restfeuchtegehalt vorzugsweise unter 15 Gew.-%, insbesondere unter 10 Gew.-% liegt, wobei der Wassergehalt gemäß der von der EDANA (European Disposables and Nonwovens Association) empfohlenen Testmethode Nr. 430.2-02 "Moisture content" bestimmt wird. Wahlweise kann zur Trocknung aber auch ein Wirbelbetttrockner oder ein beheizter Pflugscharmischer verwendet werden. Um besonders weiße Produkte zu erhalten, ist es vorteilhaft bei der Trocknung dieses Gels einen schnellen Abtransport des verdampfenden Wassers sicherzustellen. Dazu ist die Trocknertemperatur zu optimieren, die Luftzu- und -abführung muss kontrolliert erfolgen, und es ist in jedem Fall auf ausreichende Belüftung zu achten. Die Trocknung ist naturgemäß umso einfacher und das Produkt umso weißer, je höher der Feststoffgehalt des Gels ist. Bevorzugt liegt der Feststoffgehalt des Gels vor der Trocknung daher zwischen 30 und 80 Gew.-%. Besonders vorteilhaft ist die Belüftung des Trockners mit Stickstoff oder einem anderen nicht-oxidierenden Inertgas. Wahlweise kann aber auch einfach nur der Partialdruck des Sauerstoffs während der Trocknung abgesenkt werden, um oxidative Vergilbungsvorgänge zu verhindern. Im Regelfall führt aber auch eine ausreichende Belüftung und Abführung des Wasserdampfes zu einem noch akzeptablen Produkt. Vorteilhaft hinsichtlich Farbe und Produktqualität ist in der Regel eine möglichst kurze Trocknungszeit.

Das getrocknete Hydrogel wird vorzugsweise gemahlen und gesiebt, wobei zur Mahlung üblicherweise Walzenstühle, Stiftmühlen oder Schwingmühlen eingesetzt werden können. Die Partikelgröße des gesiebten, trockenen Hydrogels beträgt vorzugsweise unter 1000 µm, besonders bevorzugt unter 900 µm, ganz besonders bevorzugt unter 800 µm, und vorzugsweise über 100 µm, besonders bevorzugt über 150 µm, ganz besonders bevorzugt über 200 µm.

Ganz besonders bevorzugt ist eine Partikelgröße (Siebschnitt) von 106 bis 850 µm. Die Partikelgröße wird gemäß der von der EDANA (European Disposables and Nonwovens Association) empfohlenen Testmethode Nr. 420.2-02 "Particle size distribution" bestimmt.

Die Grundpolymere werden vorzugsweise anschließend oberflächennachvernetzt. Hierzu geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A-0 083 022, EP-A-543 303 und EP-A-937 736 beschrieben, di- oder polyfunktionelle Alkohole, wie in DE-C-33 14 019, DE-C-35 23 617 und EP-A-450 922 beschrieben, oder β-Hydroxyalkylamide, wie in DE-A-102 04 938 und US-6,239,230 beschrieben.

Des weiteren sind in DE-A-40 20 780 zyklische Karbonate, in DE-A-198 07 502 2-Oxazolidon und dessen Derivate, wie 2-Hydroxyethyl-2-oxazolidon, in DE-A-198 07 992 Bis- und Poly-2-oxazolidinone, in DE-A-198 54 573 2-Oxotetrahydro-1,3-oxazin und dessen Derivate, in DE-A-198 54 574 N-Acyl-2-Oxazolidone, in DE-A-102 04 937 zyklische Harnstoffe, in DE-A- 103 34 584 bizyklische Amidacetale, in EP-A-1 199 327 Oxetane und zyklische Harnstoffe und in WO-A-03/031482 Morpholin-2,3-dion und dessen Derivate als geeignete Oberflächennachvernetzer beschrieben.

Die Nachvernetzung wird üblicherweise so durchgeführt, dass eine Lösung des O-berflächennachvernetzers auf das Hydrogel oder das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige^{®}-Mischer, Bepex^{®}-Mischer, Nauta^{®}-Mischer, Processall^{®}-Mischer und Schugi^{®}-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex^{®}-Trockner und Nara^{®}-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250°C, bevorzugt bei 50 bis 200°C, und besonders bevorzugt bei 50 bis 150°C. Die bevorzugte Verweilzeit bei dieser Temperatur im Reaktionsmischer oder Trockner beträgt unter 30 Minuten, besonders bevorzugt unter 10 Minuten.

Das erfindungsgemäße Verfahren ermöglicht die wirtschaftliche kontinuierliche Herstellung nachvernetzter wasserabsorbierender Polymerpartikel. Die verwendeten Vernetzer werden effizient genutzt. Der Anteil an nicht umgesetzten Vernetzer und an unvernetzten Polymeren ist besonders niedrig.

### Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

### Restvernetzer

Der Gehalt an Restvernetzer der wasserabsorbierenden Polymerpartikel wird mittels HPLC unter Verwendung einer Umkehrphasensäule vom Typ ZORBAX^{®} Eclipse XDB C18 (Agilent Technologies, US) mit nachfolgender UV/VIS-Detektion und Kalibrierung mit externem Standard bestimmt. Als mobile Phase wird Acetonitril/Wasser mit einem Gradienten verwendet.

### Extrahierbare

Der Anteil an Extrahierbaren in den wasserabsorbierenden Polymerpartikeln wird gemäß der von der EDANA (European Disposables and Nonwovens Association) empfohlenen Testmethode Nr. 470.2-02 "Extractables" bestimmt.

Die EDANA-Testmethoden sind beispielsweise erhältlich bei der European Disposables and Nonwovens Association, Avenue Eugene Plasky 157, B-1030 Brüssel, Belgien.

### Beispiele:

### Beispiel 1

Durch kontinuierliches Mischen von Wasser, 50gew.-%iger Natronlauge und Acrylsäure wurde eine 38,8gew.-%ige Acrylsäure/Natriumacrylatlösung mit einem Neutralisationsgrad von 71,3 mol-% hergestellt. Die Monomerlösung wurde beim Mischen der Komponenten durch einen Wärmeaustauscher kontinuierlich auf eine Temperatur von 29°C gekühlt.

Als mehrfach ethylenisch ungesättigter Vernetzer wurde Polyethylenglykoldiacrylat (Diacrylat eines Polyethylenglykols mit einem mittleren Molgewicht von 400 g/mol) verwendet. Die Einsatzmenge betrug 2 kg pro t Monomerlösung. Der Vernetzer wurde über einen Zugabepunkt eindosiert. Die Zugabe erfolgt über eine Rohrleitung mit einem Durchmesser von 0,5 cm. Der Zugabeort des Vernetzers lag 1 m vor Reaktoreingang. Die Verweilzeit des Vernetzers in der Monomerlösung vor dem Polymerisationsreaktor betrug 1,5 Sekunden.

Nach dem Vernetzer wurden Wasserstoffperoxid und Natriumperoxodisulfat in die Monomerlösung dosiert. Die Einsatzmengen pro t Monomerlösung betrugen 1,0 kg 0,25gew.-%iges Wasserstoffperoxid und 3,1 kg 15gew.-%iges wässriges Natriumperoxodisulfat.

Der Durchsatz der Monomerlösung betrug 18 t/h.

Die Monomermischung sowie Ascorbinsäure wurden kontinuierlich in einen List Contiknet-Reaktor (Fa. List, Arisdorf, Schweiz) dosiert. Der Druck im Reaktor war gegenüber der Umgebung um 10 mbar erhöht. Die Einsatzmenge an 1gew.-%iger wässriger Ascorbinsäure betrug 1,1 kg pro t Monomerlösung.

Die Reaktionslösung wurde vor Zulauf mit Stickstoff entgast und hatte am Zulauf eine Temperatur von 23,5°C. Der Reaktor wurde mit einer Drehzahl der Wellen von 38Upm betrieben. Die Verweilzeit der Reaktionsmischung im Reaktor betrug 15 Minuten.

Nach erfolgter Polymerisation und Gelzerkleinerung wurde das Polymergel auf einen Bandtrockner aufgegeben. Während der Trocknung wurde ein gegenüber dem Umgebungsdruck um 5 mbar verringerter Druck eingestellt. Das vorzerkleinerte Polymergel wurde mit einer Schichtdicke von 10 cm auf den Bandtrockner aufgegeben und mit warmer Luft (175°C) getrocknet. Die Verweilzeit im Bandtrockner betrug 37 Minuten.

Das erhaltene Polymerpulver wurde gemahlen, gesiebt (100 bis 800 µm) und oberflächennachvernetzt.

Als Nachvernetzer wurde eine 1,2gew.-%ige Lösung von Ethylenglykoldigycidylether in Propylenglykol/Wasser (1:2) verwendet. Bezogen auf das Polymerpulver wurden 5 Gew.-% Nachvernetzerlösung aufgesprüht und 60 Minuten bei 150°C thermisch nachbehandelt.

Das nachvernetzte Polymerpulver wurde analysiert. Die Ergebnisse sind in Tabelle 1 zusammengefasst.

### Beispiel 2

Es wurde verfahren wie unter Beispiel 1. Der Zugabeort des Vernetzers lag 3,5 m vor Reaktoreingang. Die Verweilzeit des Vernetzers in der Monomerlösung vor dem Polymerisationsreaktor betrug 5,3 Sekunden.

Das nachvernetzte Polymerpulver wurde analysiert. Die Ergebnisse sind in Tabelle 1 zusammengefasst.

### Beispiel 3

Es wurde verfahren wie unter Beispiel 1. Der Zugabeort des Vernetzers lag 2,5 m vor Reaktoreingang. Die Verweilzeit des Vernetzers in der Monomerlösung vor dem Polymerisationsreaktor betrug 3,8 Sekunden.

Das nachvernetzte Polymerpulver wurde analysiert. Die Ergebnisse sind in Tabelle 1 zusammengefasst.

**Tabelle 1: Verweilzeit des Vernetzers**

| Beispiel | Verweilzeit | Extrahierbare | Restvernetzer |
|---|---|---|---|
| 1 | 1,5 s | 10,1 Gew.-% | 0,0110 Gew.-% |
| 2 | 5,3 s | 9,2 Gew.-% | 0,0070 Gew.-% |
| 3 | 3,8 s | 8,4 Gew.-% | 0,0025 Gew.-% |

Die Ergebnisse zeigen, dass Restvernetzer und Extrahierbare mit steigender Verweilzeit ein Minimum durchlaufen.

### Beispiel 4

Es wurde verfahren wie unter Beispiel 1. Der Zugabeort des Vernetzers lag 2,5 m vor Reaktoreingang. Die Verweilzeit des Vernetzers in der Monomerlösung vor dem Polymerisationsreaktor betrug 3,8 Sekunden.

Zur Dosierung des Vernetzers wurde ein 93,2 cm langes Venturi-Rohr eingesetzt (Figur 1), wobei sich die Rohrleitung über eine Strecke von 8,4 cm von einen Durchmesser von 9 cm auf 3,6 cm verjüngte (Strecke L₁), über eine Strecke von 27,6 cm den Durchmesser von 3,6 cm beibehielt (Strecke L₂) und sich über eine Strecke von 57 cm wieder von einen Durchmesser von 3,6 cm auf 9 cm weitete (Strecke L₃).

Der Vernetzer wurde über eine Rohrleitung mit einem Innendurchmesser von 5 mm in das Venturi-Rohr dosiert. Die Rohrleitung mündete 5 cm hinter dem Anfang der Einschnürzone.

Das nachvernetzte Polymerpulver wurde analysiert. Die Ergebnisse sind in Tabelle 2 zusammengefasst.

### Beispiel 5

Es wurde verfahren wie unter Beispiel 4. Der Vernetzer wurde über vier Rohrleitungen mit einem Innendurchmesser von 5 mm in das Venturi-Rohr dosiert. Die Rohrleitungen mündeten 5 bzw. 13 cm hinter dem Anfang der Einschnürzone. Die Rohrleitungen lagen sich paarweise gegenüber. Die Rohrachsen der beiden Rohrleitungspaare waren um 90° gegeneinander gedreht.

Das nachvernetzte Polymerpulver wurde analysiert. Die Ergebnisse sind in Tabelle 2 zusammengefasst.

### Beispiel 6

Es wurde verfahren wie unter Beispiel 4. Der Vernetzer wurde über acht Rohrleitungen mit einem Innendurchmesser von 5 mm in das Venturi-Rohr dosiert. Die Rohrleitungen mündeten 5 bzw. 13 cm hinter dem Anfang der Einschnürzone, wobei jeweils vier Rohrleitungen senkrecht aufeinander standen.

**Tabelle 2: Anzahl der Zuführungen**

| Beispiel | Anzahl der Zuführungen | Extrahierbare | Restvernetzer |
|---|---|---|---|
| 4 | 1 | 8,0 Gew.-% | 0,0015 Gew.-% |
| 5 | 4 | 8,3 Gew.-% | 0,0009 Gew.-% |
| 6 | 8 | 8,4 Gew.-% | <0,0008 Gew.-% |

Die Ergebnisse zeigen, dass mit steigender Zahl der Zuführungen der Restvernetzer abnimmt.

## Patentansprüche

1. Verfahren zur kontinuierlichen Herstellung wasserabsorbierender Polymere, wobei eine Monomerlösung mit mindestens einem Vernetzer vermischt und die erhaltene Mischung polymerisiert wird, **dadurch gekennzeichnet, dass** die Verweilzeit der Mischung zwischen der Zugabe des mindestens einen Vernetzers und dem Eintritt in den Polymerisationsreaktor mindestens eine Sekunde und weniger als 60 Sekunden beträgt.

2. Verfahren gemäß Anspruch 1, **dadurch gekennzeichnet, dass** die Verweilzeit der Mischung zwischen der Zugabe des mindestens einen Vernetzers und dem Eintritt in den Polymerisationsreaktor weniger als 10 Sekunden beträgt.

3. Verfahren gemäß Anspruch 1 oder 2, **dadurch gekennzeichnet, dass** die innere Oberfläche der Verbindung zwischen der Zuführung des mindestens einen Vernetzers und Polymerisationsreaktor zumindest teilweise gegenüber Wasser einen Kontaktwinkel von mindestens 60° aufweist.

4. Verfahren gemäß einem der Ansprüche 1 bis 3, **dadurch gekennzeichnet, dass** der mindestens eine Vernetzer in der Mischung nicht vollständig löslich ist.

5. Verfahren gemäß einem der Ansprüche 1 bis 4, **dadurch gekennzeichnet, dass** die Mischung inertisiert wird.

6. Verfahren gemäß einem der Ansprüche 1 bis 5, **dadurch gekennzeichnet, dass** der mindestens eine Vernetzer über ein Venturi-Rohr in die Monomerlösung dosiert wird.

7. Verfahren gemäß einem der Ansprüche 1 bis 6, **dadurch gekennzeichnet, dass** die Mischung zwischen der Zugabe des mindestens einen Vernetzers und der Polymerisation zumindest teilweise mit einer Geschwindigkeit strömt, die einer Reynolds-Zahl von 1000 bis 10.000 entspricht.

8. Verfahren gemäß einem der Ansprüche 1 bis 7, **dadurch gekennzeichnet, dass** mindestens 50 mol-% der Monomere der Monomerlösung Acrylsäure und/oder deren Salze sind.

9. Verfahren gemäß einem der Ansprüche 1 bis 8, **dadurch gekennzeichnet, dass** die Monomerlösung im Polymerisationsreaktor zu einem Hydrogel polymerisiert, getrocknet, gemahlen und klassiert wird.

10. Verfahren gemäß Anspruch 9, **dadurch gekennzeichnet, dass** die klassierten Polymerpartikel oberflächennachvernetzt werden.

## Claims

1. A process for continuously preparing water-absorbing polymers by mixing a monomer solution with at least one crosslinker and polymerizing the resulting mixture, wherein the residence time of the mixture between the addition of the at least one crosslinker and the entry into the polymerization reactor is at least one second and less than 60 seconds.

2. The process according to claim 1, wherein the residence time of the mixture between the addition of the at least one crosslinker and the entry into the polymerization reactor is less than 10 seconds.

3. The process according to claim 1 or 2, wherein the inner surface of the connection between the feed of the at least one crosslinker and polymerization reactor at least partly has a contact angle for water of at least 60°.

4. The process according to any of claims 1 to 3, wherein the at least one crosslinker is not completely soluble in the mixture.

5. The process according to any of claims 1 to 4, wherein the mixture is inertized.

6. The process according to any of claims 1 to 5, wherein the at least one crosslinker is metered into the monomer solution via a Venturi tube.

7. The process according to any of claims 1 to 6, wherein the mixture, between the addition of the at least one crosslinker and the polymerization, flows at least partly with a velocity which corresponds to a Reynolds number of from 1000 to 10 000.

8. The process according to any of claims 1 to 7, wherein at least 50 mol% of the monomers of the monomer solution are acrylic acid and/or salts thereof.

9. The process according to any of claims 1 to 8, wherein the monomer solution is polymerized in the polymerization reactor to give a hydrogel, dried, ground and classified.

10. The process according to claim 9, wherein the classified polymer particles are surface postcrosslinke

## Revendications

1. Procédé de préparation continue de polymères absorbant l'eau, selon lequel une solution de monomères est mélangée avec au moins un agent de réticulation et le mélange obtenu est polymérisé, **caractérisé en ce que** le temps de séjour du mélange entre l'ajout du ou des agents de réticulation et l'entrée dans le réacteur de polymérisation est d'au moins une seconde et de moins de 60 secondes.

2. Procédé selon la revendication 1, **caractérisé en ce que** le temps de séjour du mélange entre l'ajout du ou des agents de réticulation et l'entrée dans le réacteur de polymérisation est de moins de 10 secondes.

3. Procédé selon la revendication 1 ou 2, **caractérisé en ce que** la surface intérieure du raccordement entre l'alimentation du ou des agents de réticulation et le réacteur de polymérisation présente au moins en partie un angle de contact avec l'eau d'au moins 60°.

4. Procédé selon l'une quelconque des revendications 1 à 3, **caractérisé en ce que** le ou les agents de réticulation ne sont pas entièrement solubles dans le mélange.

5. Procédé selon l'une quelconque des revendications 1 à 4, **caractérisé en ce que** le mélange est inertisé.

6. Procédé selon l'une quelconque des revendications 1 à 5, **caractérisé en ce que** le ou les agents de réticulation sont dosés dans la solution de monomères par un tube de Venturi.

7. Procédé selon l'une quelconque des revendications 1 à 6, **caractérisé en ce que** le mélange s'écoule au moins en partie à une vitesse correspondant à un nombre de Reynolds de 1 000 à 10 000 entre l'ajout du ou des agents de réticulation et la polymérisation.

8. Procédé selon l'une quelconque des revendications 1 à 7, **caractérisé en ce qu'**au moins 50 % en moles des monomères de la solution de monomères sont de l'acide acrylique et/ou ses sels.

9. Procédé selon l'une quelconque des revendications 1 à 8, **caractérisé en ce que** la solution de monomères est polymérisée dans le réacteur de polymérisation en un hydrogel, séchée, broyée et classifiée.

10. Procédé selon la revendication 9, **caractérisé en ce que** les particules polymères classifiée sont post-réticulées en surface.
